(19) Europäisches Patentamt
European Patent Office
Office européen des brevets

(11) **EP 4 067 547 A1**

(12) **EUROPEAN PATENT APPLICATION**

(43) Date of publication:
**05.10.2022 Bulletin 2022/40**

(21) Application number: **21166826.4**

(22) Date of filing: **02.04.2021**

(51) International Patent Classification (IPC):
**D04H 1/4274** (2012.01)    **D04H 1/435** (2012.01)
**D01F 6/62** (2006.01)    **A61F 13/49** (2006.01)

(52) Cooperative Patent Classification (CPC):
**D04H 1/4274; A61F 13/49; D01D 1/04; D01F 6/62;
D04H 1/435;** D01D 5/26

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **TWE Meulebeke**
**8760 Meulebeke (BE)**

(72) Inventors:
• **MICHIELS, Dany**
**9450 Haaltert (BE)**
• **CHRISTIAEN, Hugo**
**9960 Assenede (BE)**
• **DEWITTE, Greet**
**8700 Tielt (BE)**
• **DECAMBRAY, Véronique**
**8791 Waregem (BE)**

(74) Representative: **Office Kirkpatrick**
**Avenue Wolfers, 32**
**1310 La Hulpe (BE)**

(54) **METHOD TO RECYCLE POLYESTER-BASED NONWOVEN TO STAPLE FIBER**

(57)    Contrary to packagings or bottles made of pure materials like PET, nonwovens usually consist of a mix of materials and are as such difficult to include in a recycling process. The present invention relates to a method for recycling polyester-based nonwovens comprising 5 to 50% of PET/coPET low melt staple fibers into polyester staple fibers (PSF), and further including these fibers into new nonwovens for a large variety of articles for applications in virtually any technical field.

Figure 1

## Description

### Field of the invention

The present invention relates to a method for recycling polyester based nonwoven comprising 5 to 50% of sheath/core or side by side or others PET and/or coPET low melt fibers into new fibers.

### Background of the invention

Polyethylene terephthalate, commonly abbreviated PET, is the most common thermoplastic polymer resin of the polyester family and is used in fibres or yarn for clothing or nonwovens, containers for liquids and foods, and thermoforming for manufacturing, and in combination with glass fibre for engineering resins.

It is known to recycle PET food containers, mainly bottles into pellets, flakes or chips to manufacture fibers for use in nonwoven materials. PET bottles being made in virtually pure PET homopolymer, the properties of the fibers obtained after recycling are only slightly modified compared to virgin PET fibers, which make them easy to use further.

This is not the case for other sources of PET. In particular, in the nonwoven industry, PET homopolymer is associated with PET copolymers (coPET), either as a mix of fibers, or even as a mix within a single fiber, in a core-sheath association. For example, thermobonded drylaid nonwovens contain 5 to 60% of low melt PET/coPET fibers as binder. A single article, for example hygiene articles, further combines several different layers of nonwovens.

The ratio between the PET/coPET being variable, the outcome of a product obtained from recycling nonwoven waste has therefore variable/unpredictable properties, and usually a lower viscosity and a lower melting temperature than virgin (not recycled) material.

Nonetheless, the applicant wishes to address the problem of recycling nonwoven waste, which can be of two types: post-industrial waste or post-consumer waste. Post-industrial waste originates mostly from the nonwoven production process or the processes to include nonwoven in further article. Post-consumer waste designates purified, cleaned or washed waste from the end-consumer. To reduce to a maximum the ecological impact of the recycling of nonwoven, it is wished to reach circularity, i.e. recycling into new fibers to make nonwovens, preferably onsite or with limited transport.

### Summary of the invention

To this end, the invention concerns a method for recycling polyester based nonwovens comprising 5 to 60% of PET/coPET low melt staple fibers comprising the steps of:

- Feeding a polyester based nonwoven to recycle into a repelletising machine to obtain recycled polyester-based pellets,
- Blend the obtained pellets with polyester flakes, the blend comprising 15 to 35% of recycled polyester-based pellets and 65 to 85% of polyester flakes, and
- Extrude the blend into polyester staple fibers (PSF).

The polyester flakes can be "virgin" polyester flakes (or chips) but, preferably, the polyester flakes originate from recycled (post industrial) PET bottles.

Polyester intends to designate any kind of polyester and derivates (PBT, PTT...), i.e. aliphatic homopolyesters or copolyesters as well as aromatic homopolyesters or copolyesters, or mixtures thereof, whatever the sources of polyesters, including recycled polyester and polyester from said green sources.

The polyester based nonwovens to be recycled can come in various form, depending on their origins. They can be for example pressed baled shredded nonwoven, over pressed baled nonwoven trims, non-pressed trims or off-spec rolls.

Low melt staple fibers (LMF) designate fibers having at least a sheath component melting at low temperature (i.e. melting temperature of around 180 °C or less, preferably melting temperature of around 160 °C or less, preferably melting temperature of around 130 °C or less and still preferably melting temperature of around 110 °C or less), and which serve as binder for other fibers having higher melting points, in the nonwoven manufacturing process. Low melt staple fibers usually have a titer of 1 dn to 30dn but can also have a larger titer. LMF usually have a core/sheath construction, but this is not mandatory.

The polyester staple fibers (PSF) obtained by the method of the invention are regular PSF, with titers varying from 0.5 dn to 40 dn, preferably 1.5 dn to 15 dn. These PSF contain between 0.75 and 17.5% of PET and/or coPET (originating from the recycled nonwoven) . Preferably, these PSF fibers have a melting point above 180°C, preferably above 200°C, still preferably above 240°C and possibly between 245°C and 255 °C.

The polyester staple fibers obtained by the method of the invention, i.e. comprising at least 0.75% of PET and/or CoPET originating from recycled nonwovens, can be distinguished from regular polyester staple fibers by a lower intrinsic viscosity of below 0.7 dl/g, preferably between 0.6 and 0.7 dl/g, like for example between 0.65 and 0.70 dl/g for white fibers, while it could be lower for black fibers, like for example around 0.62 dl/g.

Advantageously, the polyester staple fibers (PSF) obtained by the method of the invention are further used to produce nonwovens, preferably 100% polyester-based-nonwovens, and still preferably, 100% polyester based staple fibers nonwovens. So, a further step of the method of the invention can be:

-    incorporate the PSF into a web of fibers for manufacturing a nonwoven material.

Further steps can read:

-    use the nonwoven material in an article, like for example a hygiene article, an absorbent article, an article for the automotive industry, the building industry, construction, filtration, medical devices, living, ...

Depending on the application, the weight percentage of PSF obtained by the method of the invention in a nonwoven can vary between 5% and 100% of the nonwoven, preferably between 10% and 98%, preferably between 15% and 95%. For example, nonwoven for mattress applications typically comprises about 15% of low melt staple fibers and can comprise up to 85% of PSF, while nonwoven for hygienic applications like acquisition and distribution layers (ADL) in baby diapers typically comprise about 50% of LMF and can comprise up to around 50% of PSF.

The invention also encompasses a nonwoven material comprising 5 to 100% by weight of the PSF comprising at least 0.75% of PET and/or CoPET originating from recycled nonwovens, having an intrinsic viscosity (IV) of below 0.7 dl/g (limiting viscosity number measured as disclosed below), the nonwoven having an opacity comprised between 20% and 50% measured according to the method described below, preferably between 25% and 40%.

The invention also concerns absorbent articles incorporating nonwoven materials of the invention. The absorbent article comprises a topsheet forming a wearer-facing surface of the absorbent article, a backsheet and an absorbent core and/or an acquisition and distribution layer (ADL) interposed between the topsheet and the backsheet, wherein the absorbent article comprises a nonwoven material, said nonwoven material comprising between 5% and 100% by weight of PSF comprising at least 0.75% of PET and/or CoPET originating from recycled nonwovens, having an intrinsic viscosity (IV) of below 0.7 dl/g (limiting viscosity number measured as disclosed below).

Advantageously, the nonwoven in the absorbent article comprises at least 20% by weight core/sheath or side by side bicomponent fibers, as binder fibers. This is particularly interesting when the manufacturing process involve a heating step.

Any one or several layers of the absorbent article can be a nonwoven according to the invention. Preferably, the nonwoven is comprised at least in the ADL. The nonwoven can also be comprised in the core, as a monolayer, dual layer or triple layer within the core.

The method of the invention can include a step of mixing a coloring dye either before the repelletizing step or before the extrusion step.

The extrusion of the blend can lead to PSF having a solid cross-section or a hollow cross-section, with some void areas. They can have a cross section of any shape, like for example but not limitingly a circular cross section, a triangular cross section, a trilobal or multi-lobal cross section,...

The PSF obtained preferably but not limitingly have a length of 20 mm or longer.

The invention will be better understood with the following description of several examples, referring to the accompanying drawing on which:

figure 1 is a general scheme of the method of the invention,

figure 2 illustrates the manufacture of a nonwoven using the recycled fibers of the invention,

figure 3 is a DSC scan of the fiber in example 1 of table 1, and figure 4 illustrates an ADL production line.

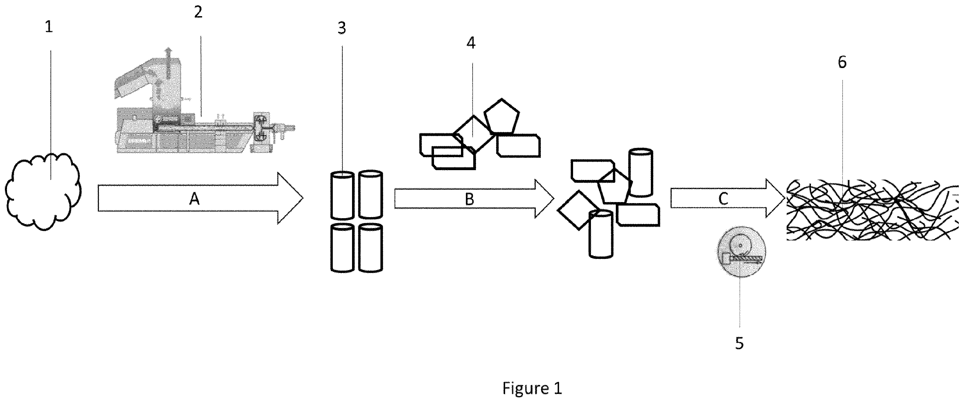

Referring to figure 1, in a step A, polyester based nonwoven material 1, is fed into a repelletizing machine 2 to obtain recycled polyester-based pellets 3. For example, the nonwoven material is here waste trims from a nonwoven manufacturing line. It comprises 5 to 50% of PET/coPET low melt staple fibers. Such a waste is usually clean and does not need to be washed before recycling. For other origin of nonwoven waste, it is possible that a preliminary washing step is required.

Repelletising machines are well known in the art of plasturgy. The principle is that it melts the nonwoven waste to its liquid state in two phases. In a first a preconditioning phase, the polymer is demoisturized and the spinning oil is removed, then the liquid polymer is degazed and then the temperature is decreased while imparting a cylindrical or spherical shape to the polymer in order to form polyester granules or pellets 3.

The intrinsic viscosity (IV) value of the obtained granules are typically lower than normal PET flake resin, because of the presence of significant percentages of LMF.

Typical IV values found will be around 0,49 to 0,60 dl/g, while IV values of PET flakes from bottles is around 0,70 to 0,75 dl/g. The IV required for spinning staple fibers is between 0.65 and 0.70 dl/g for white fibers, while it could be lower for black fibers, like for example around 0.62 dl/g.

A possibility to increase the IV known in the art would be to add a further SSP (solid state polymerization) step after the repelletising, but this would increase significantly the cost price of the process. The method of the invention is therefore preferably devoid a SSP step.

The melting temperature is an alternative characterizing value of the polymer. While normal PET has a melting temperature of around between 260 and 265 °C, the pellets 3 have a melting temperature of several degrees lower, i.e. between 253 and 258°C, so on average 7°C lower for the recycled material compared to the regular material.

Several repelletising machine manufacturers exist, like Erema, Starlinger, Moogetech,... there is no limitation here whatsoever for using a specific machinery technology.

Because the pellets 3 have an intrinsic viscosity slightly too low to be directly extruded into staple fibers, in a step B, the pellets 3 blended with polyester flakes 4, coming from recycled PET bottle. The blend comprises 15 to 35% of recycled polyester-based pellets 3 and 65 to 85% of polyester flakes 4.

The ratio of pellets 3 versus flakes 4 is preferably adjusted after measuring the IV and/or the melting temperature of the pellets 3. The higher this temperature or IV, the larger percentage of pellets can be added into the blend.

The blend is for example made in an Oerlikon Barmag mixer, or Neumag equipment, or any suitable equipment known to the person skilled in the art.

In a step C, the blend enters an extrusion unit 5 where it is extruded into polyester staple fibers (PSF). The extrusion step is classical of textile fiber production and well known to the person skilled in the art, and can be for example a two steps production method, also known as long spin method, or a single step production method, also known as short spin method.

The ratio in the blend, determined in function of the characteristics of the pellets has some impact on the characteristics of the final PSF, i.e. the tenacity, elongation, shrinkage levels, ... of the fibers. It is important for the subsequent process of integration of the PSF into nonwoven that the PSF issued of the method of the invention fall within a "virgin" (not incorporating recycled materials) PSF type Technical Datasheet.

Examples of technical characteristics obtained for virgin fibers compared to recycled fibers are given in Table 1 below.

Table 1

| | Example 1 | Example 2 | Example 3 | Example 4 | Example 5 | Example 6 | Comparative example |
|---|---|---|---|---|---|---|---|
| Origin Nonwovens Waste (weight % LMF in waste) | 50 | 50 | 50 | 15 | 15 | 15 | 0 |
| Intrinsic Viscosity (IV) in $cm^3$/g of PET waste pellets (LVN) | 58,0 | 57,6 | 56,0 | 54,8 | 48,9 | 54,0 | >70 |
| Melt flow rate (MFR) in $cm^3$/10 min of PET waste pellets | | | 88,789 | | | 101,611 | |
| Melting point in °C of PET waste pellets | 255 | 255 | 253,5 | 255 | 255 | 253,5 | 260 |
| Weight % PET waste pellets | 22 | 22 | 22 | 18 | 18 | 18 | 0 |
| % regular PET flakes | 78 | 78 | 78 | 82 | 82 | 82 | 100 |
| Repelletizing Machine used | EREMA | EREMA | STARLINGER | EREMA | EREMA | STARLINGER | N/A |
| Intrinsic Viscosity of the final PSF in $cm^3$/g | 63,1 | 60,4 | 69,59 | 67,98 | 65,64 | 70,49 | |
| Melting point in °C of the final PSF | 249 | 249 | 249 | 251 | 251 | 251 | 256 to 258 |
| titer in dtex | 6,56 | 6,93 | 6,52 | 6,4 | 6,73 | 6,61 | 6,46 |
| titer standard deviation | | 0,83 | 0,61 | 0,52 | | 0,47 | 0,79 |
| Tensile strength in cN/dtex | 2,99 | 2,98 | 2,94 | 3,14 | 3,15 | 3,16 | 3,48 |
| Tensile strength standard deviation | | 0,43 | 0,22 | 0,41 | | 0,35 | 0,34 |
| Elongation in % | 96,32 | 93,08 | 87,5 | 91,96 | 95,48 | 96,69 | 72,31 |
| Elongation standard Deviation | | 15,02 | 22,35 | 20,3 | | 10,94 | 15,3 |
| Shrinkage in % in hot air 30 minutes at 160°C | 0,87 | 0,93 | 0,67 | 0,6 | 0,87 | 0,87 | 1,27 |
| nr of crimps/inch | 7,75 | 8,25 | 7,7 | 7,95 | 8,6 | 8,9 | 8,3 |
| Cut length in mm | 60,1 | 59,2 | 60,3 | 60,2 | 61,1 | 61 | 61,8 |
| L Colour value | 89,37 | 89,67 | 91,66 | 91,27 | 91,08 | 90,77 | 90,98 |
| a Colour Value | -0,09 | -0,54 | -1,4 | -1,15 | -1,16 | -0,86 | -1,21 |
| b Colour Value | 0,77 | 1,66 | 2,2 | 2,35 | 2,76 | 1,58 | 2,85 |
| w whiteness Value | 78,61 | 67,67 | 69,59 | 67,98 | 65,64 | 70,49 | 64,99 |

EP 4 067 547 A1

**Intrinsic Viscosity** (IV) here refers to the limiting viscosity number (LVN, intrinsic viscosity, I.V., $[\eta]$) of polyesters based on polyethylene terephthalate (PET) in form of fibres, granulates, regranulates, preforms, foils, flakes, black, white and colour masterbatches (can be based on PBT).in a Viscosity meter Ubbelohde U 1a connected to an Automatic Lauda equipment to measure flow times of the sample solution and solvent, using the following procedure:

a) Solvent preparation - cleaning

The mixture used for measurement is made of phenol (CAS: 108-95-2) and 1,1,2,2- tetrachloroethane (CAS: 79-34-5), using the weight ratio 1 : 3.
Phenol is distilled with an air cooler and the fraction at 182 °C is collected.
1,1,2,2- tetrachloroethane is distilled with an air cooler at ca. 140 °C. Concentrated solution of soda (ca. 20 ml per 1 litre) is added to the distillation receiver in the flask. The fraction with the boiling point 144 - 145 °C is collected.

b) Measurement

The polyester samples shall be completely dry. Remove any finish from the polyester fibre sample by thorough washing in distilled water and then dry it completely. Black masterbatch is milled before weighing.
Weigh out 0.2500 g of the sample, place it into the flask and add 25 ml of the solvent from the burette; let the sample dissolve in the drying oven at 105 °C (maximally 2 hours, for fiber sample 30 minutes should be enough). Agitate the flask content every 5 minutes. After visually checking that the sample has been dissolved wait 10 more minutes. After the solution cools down use a sintered glass S2 to filter it into the viscosity meter; use the first part of the filtrate to flush the viscosity meter. Suspend the viscosity meter filled with the sample solution in a thermostat bath and after 10 minutes of heating at 30 °C measure the flow time of the solution 4 times. Before measuring the sample measure the flow time of the pure solvent first (average from 3 measurements, temperation 10 minutes, 30°C). Perform Hagenbach kinetic energy correction of the measured times for the solution and for the pure solvent:

$$t = tm - B/A.tm$$

A .......... capillary constant (0.03)
B .......... viscosity meter constant (2.8)
tm ........ measured flow time (s)

c) Test evaluation

Calculate the limiting viscosity number from the relative viscosity at the concentration
c = 1 g / 100ml using the Huggins equation. The Huggins constant for PET under the given measuring conditions is k = 0.25.
The limiting viscosity number (LVN) in ml.g$^{-1}$ is then calculated using the following formula (which applies only to the employed experimental condition)with:

t : corrected flow time of the sample
$t_o$ : corrected flow time of the pure solvent

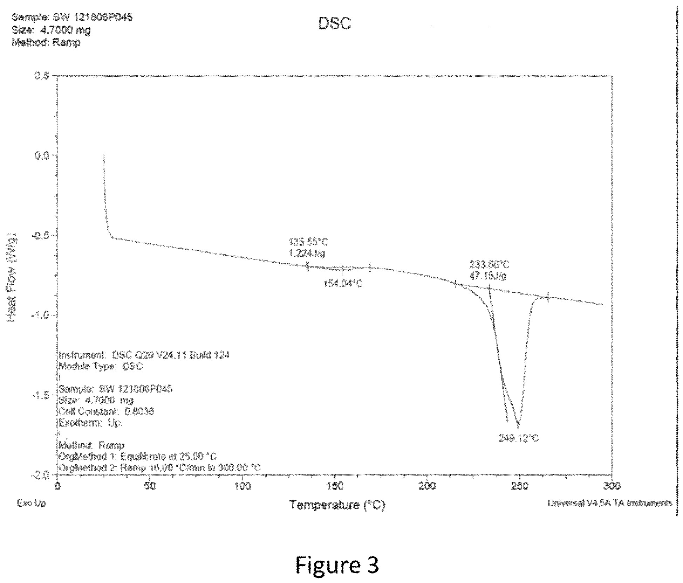

The Lauda viscosity meter with a connected computer performs all computations automatically, including measurement of the flow times and corrections. The laboratory worker only reads the LVN value.
**Melt flow rate** (MFR) was measured according to the ASTM D1238, ISO 1133 standard method.
**The melting point** was measured according by DSC, as usually done in the field, using for example a stabilization phase at 25 °C followed by a temperature ramp of 16.00 °C/min up to 300 °C. Figure 3 illustrates the DSC scan of the fiber of example 1 in Table 1.
The **titer** was measured using a VIBROSKOP instrument made by Lenzing Instruments. Individual fibres with a defined pretension are placed into the device that performs automatic measurements using the vibration principle. Twenty individual fibres are measured and the resulting value is their average.
The **tensile strength (tenacity) and elongation** were measured using a VIBRODYN instrument made by Lenzing Instruments. Individual fibres are fixed into tearing jaws and exposed to mechanical stress at a defined speed and clamping length. A defined force pulls the fibre until it breaks. The resulting elongation percentage at the moment it breaks is calculated as well as the total force applied to the titer ratio. Twenty individual fibres are measured and the resulting value is their average.
The **shrinkage** was measured in hot air at a temperature of 160 °C for 30 minutes. Shrinkage is defined as difference in length before and after fibre fixing in percents. 5 tows of 50 cm long are measured.

The **nr of crimps/inch** was measured using a standardized meter at a defined pretension. Individual fibres are fixed to a measuring base plate and the number of crimps is read visually. Twenty individual fibres are measured and the resulting value is their average.

The **colour values** was measured on a reflex spectrophotometer DT600 made by Datacolor. The measurement uses especially prepared samples and an accurately defined setup of the device at different types and wavelengths of light. The reflection principle is used to determine basic color coordinates (L, a, b). Three samples are measured and the resulting value is their average. Fibers are combed and arranged in a layer which is inserted in the spectrophotometer for read-out. The basic color coordinates (L, a, b) are extracted from the read-out.

It was generally observed that the color values of the PSF obtained by the method of the invention are slightly darker and have a stronger yellow component than "virgin" fibers.

The PSF obtained by the method of the invention can be incorporated in any kind of nonwoven, just as "virgin" fibers.

For example, a polyester based thermobonded drylaid nonwoven can be manufactured using 50% of PSF fibers obtained by the recycling method of the invention and 50% of Low Melt Fibers for incorporation as ADL (absorption and distribution layer) in a hygiene article.

For example, a polyester based thermobonded drylaid nonwoven can be manufactured using 50 % of PSF fibers obtained by the recycling method of the invention, 35 % of standard PSF and 15% of Low Melt Fibers for fiberfill applications.

Test 1 - Thermobonded nonwoven material for use in bedding / quilting / furniture applications.

A thermobonded nonwoven material according to the invention was manufactured for use in bedding / quilting / furniture applications.

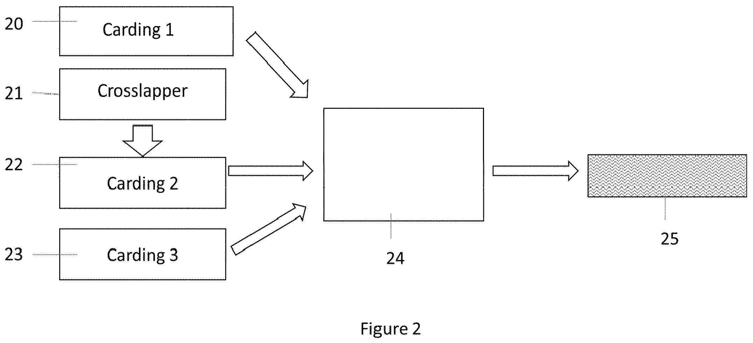

Referring to Figure 2, a nonwoven 25 was manufactured according to a standard process, starting from three carding machines 20, 22, and 23. A crosslapper 21 is here implemented downstream of carding machine 22. Blending units (not represented) are installed upstream of the carding machine to blend the fibers used for each web.

Carding is a mechanical process that disentangles, cleans and intermixes fibres to produce a continuous web suitable for subsequent processing. This is achieved by passing the fibers between differentially moving surfaces covered with card clothing. It breaks up locks and unorganized clumps of fibers and then aligns the individual fibers to be parallel with each other. The fibers not being straight elements, they are not strictly parallel, but globally orientated in a common general direction. The fact that the fibers are not straight elements enables some contact points between fibers, these contact points being the bonding points during the bonding step.

Depending on the thickness and/or weight expected for the nonwoven material, several layers of carded fibers, of a same or of different compositions, can be overlaid before bonding, using techniques and equipment well known to a person skilled in the art. Using several carding machines in parallel, usually up to three, allows to work at high speed. The resulting webs are then overlapped before webbonding, which can be mechanical bonding, thermobonding and/or chemical bonding. This also presents the advantage of being able to combine the different properties of several fiber blends.

In this inline configuration, the overall thickness of the nonwoven depends on the thickness of the web issued from each carding machine and of the number of carding machines operating in parallel. In general, no more than three parallel carding machines are used, limiting the weight of the resulting nonwoven to about 200 g/m$^2$, preferably 120 g/m$^2$, though these figures are not limiting. Moreover, the general orientation conferred to the fibers in the carding step is maintained in the bonding step, resulting in a nonwoven having a high longitudinal resistance to tear, longitudinal meaning parallel to the general orientation of the fibers, and a lower lateral resistance to tear.

However, a crosslapping step can be implemented on one or several of the carding lines after carding, leading to overlapping several layers of web of carded fibers, and additionally imprinting a deviation in the general orientation of the fibers, which is different between two consecutive overlap. The crosslapping therefore results in a much thicker nonwoven material than when using the inline process, typically over 120 g/m$^2$. The resulting nonwoven is also more resistance to tear in all directions.

The webs obtained from the three carding machines are then combined on top of each other into a three-way oven 24 in order to form a single thermobonded nonwoven 25.

An optional step of needling can be implemented downstream of carding and overlapping, before heating the overlapped webs, resulting in entangling or mixing up the fibers in the vertical dimension. This is especially recommended when more than one carding machine are used. The vertical dimension here refers to a direction perpendicular to the longitudinal and lateral dimensions disclosed in the previous paragraphs, i.e. a direction crossing the various layers of webs. Needling enables to obtain a better adhesion of the web layers, by entangling the fibers. Hydroentanglement could also be used instead of or additionally to needling. Needling and hydroentangling are techniques well known from the person skilled in the art. Other reinforcing techniques can also be used like, for example, chemical bonding, either by extrusion of a polymer, knife over roll or any suitable coating technique, as known to the person skilled in the art.

As a final step, after bonding, the nonwoven undergoes winding and packaging.

Bonding of the fibers to finalize the nonwoven layer is here performed by thermobonding, but it can also be performed using different techniques, like mechanical or chemical bonding. In the present case, bonding preferably includes a step of thermal bonding, either alone, or in combination with another technique. Preferably, the nonwoven of the invention is a drylaid thermobonded nonwoven. A combination of mechanical, chemical and thermo- bonding is also an option.

The blend of fibers provided to the second carding machine 22 is here a blend comprising:

- 25% bicomponent fiber PET/CoPET 4dn - 51 mm
- 65% of PSF fibers obtained from recycling and corresponding to a mix of the fibers of examples 1 to 6of Table 1 (6dn - 60 mm)
- 10% internal waste (fibers recollected from the edges of the web when dimensioning the web to proper breadth, before the binding step, and reincorporated in the blend).

The blend of fibers provided to the carding machines 20 and 23 is here a blend comprising:

- 25% bicomponent fiber PET/CoPET 4dn - 51 mm
- 30% solid PET fibers 6dn - 64 mm
- 30% of PSF fibers obtained from recycling and corresponding to a mix of the fibers of examples 1 to 6of Table 1 (6dn - 60 mm)
- 15% internal waste (fibers recollected from the edges of the web when dimensioning the web to proper breadth, before the binding step, and reincorporated in the blend).

The carding machines 22 and 23 provide webs counting each for 30% of the final product, while carding machine 20 provides a web counting for 40% of the final nonwoven. As a result, the final nonwoven comprises 44% of recycled PSF according to the invention. The obtained nonwoven has a weight per surface area of about 150 g/m$^2$.

Four trials were made. A sample of the nonwoven for each trial has been characterized for their opacity using the following the ASTM method, and compared to the equivalent sample made from non recycled fibers (Thermoloft 21).

Opacity is determined by the reflectance obtained from the material with a black background, as well as the reflectance obtained for the same material with a white background.

The equipment used are:

- Hunter ColorFlex EZ Spectrophotometer (sensor number: ColorFlex EZ 45/0 "CFEZ 3350")
- EasyMatch QC software
- Colorflex EZ Standard Box :

  ☐ White and black tile for calibration
  ☐ Green tile for an extra check
  ☐ Black and white tile to perform a test.

The apparatus is standardized according to manufacturer's instructions, using the white, black and green tiles provided in the Colorflex EZ Standard box.

The settings are adjusted to :

- illuminant D65/10
- Observer 10°
  125x125mm samples of nonwovens are cut and the test can be performed according to the following procedure:
- place the sample on the device on the measuring cell. Machine direction (MD) of the sample is in line with the device and the smooth side of the sample is facing down.
- place the white tile on the sample (with the white circle downwards);
- measure with the white tile;
- remove white tile and replace with the black tile (with the white circle downwards);
- measure with the black tile;
- the opacity is now calculated and displayed as the percent ratio of the reading with the black plate over the reading with white plate.

The opacity is measured for 5 cuts of each sample and the averages are given below:

Sample 1: 79.45%
Sample 2: 76.53%
Sample 3: 77.90%
Sample 4: 79.62%
Thermoloft 21: 80.77%.

The same four samples have been characterized for their dynamic loading measured following the pounding test according to ISO 3385. The results are gathered in following table 2.

Table 2

| | static load in g/cm$^2$ : | 0 | 0,6 | 2,44 | 4 | 10 | 19,5 | 30 |
|---|---|---|---|---|---|---|---|---|
| Sample 1 (156.08 g/m$^2$ on average) | init(mm) | 22,28 | 20,26 | 16, 95 | 14,50 | 8, 97 | 5,58 | 4,01 |
| | final (mm) | 11,84 | 9,87 | 7,23 | 5,76 | 3,45 | 2,42 | 2, 02 |
| | **point %** | **53,13** | **48,69** | **42, 62** | **39,69** | **38,45** | **43,34** | **50,44** |
| Sample 2 (157.78 g/m$^2$ on average) | Init (mm) | 19, 62 | 18, 01 | 15,58 | 13,86 | 9,13 | 5,96 | 4,27 |
| | final (mm) | 12,33 | 10,48 | 7,99 | 6,62 | 3,69 | 2,75 | 2,22 |
| | **point %** | **62,87** | **58,21** | **51,32** | **47,76** | **40,44** | **46,21** | **52,05** |
| Sample 3 (156.45 g/m$^2$ on average) | init(mm) | 20,89 | 19,32 | 15,86 | 13,59 | 8,71 | 5,64 | 4,02 |
| | final (mm) | 14,34 | 11,15 | 7,93 | 6,30 | 3,68 | 2,61 | 2,16 |
| | **point %** | **68,63** | **57,70** | **50,01** | **46,35** | **42,28** | **46,22** | **53,75** |
| Sample 4 (159.03 g/m$^2$ on average) | init(mm) | 19,42 | 18,02 | 15,00 | 13,44 | 9,12 | 5,73 | 4,07 |
| | final (mm) | 13,05 | 11,04 | 7,95 | 6,36 | 4,02 | 2,78 | 2,28 |
| | **point %** | **67,20** | **61,27** | **53,01** | **47,34** | **44,10** | **48,42** | **55,85** |
| Thermoloft 21 (150 g/m$^2$ ) | init(mm) | 24,26 | 22,02 | 19,37 | 16,99 | 10,58 | 6,53 | 4,53 |
| | final (mm) | 16,01 | 14,26 | 10,27 | 8,07 | 4,32 | 2,78 | 2,22 |
| | **point %** | **66,02** | **64,76** | **52,99** | **47,48** | **40,87** | **42,59** | **49,06** |

The same four samples have been characterized for their elongation and tensile strength both in the machine direction (MD) and carding direction (CD), measured following the test according to ISO 9073-3. The results are gathered in following table 3.

Table 3.

| | | Tensile strength (N/10cm) | Elongation at Fmax (%) |
|---|---|---|---|
| sample 1 | MD | 53,90 | 38,20 |
| | CD | 27,26 | 58,65 |
| sample 2 | MD | 60,70 | 38,46 |
| | CD | 31,06 | 62,38 |
| sample 3 | MD | 50,13 | 39,94 |
| | CD | 30,60 | 55,76 |
| sample 4 | MD | 53,98 | 40,26 |
| | CD | 27,73 | 64,65 |
| Thermoloft 21 | MD | 49,56 | 35,87 |
| | CD | 28,22 | 57,89 |

The results of table 2 and 3 demonstrate the equivalence between the reference nonwoven not including recycled fibers and the nonwoven according to the invention.

In a similar manner, nonwovens having a weight per surface area of about 300 g/m² have been manufactures and compared to the equivalent Thermoloft 21 reference.

The results are gathered in Table 4 and 5.

Table 4.

| | static load in g/cm² | 0 | 0,6 | 2,44 | 4 | 10 | 19,5 | 30 |
|---|---|---|---|---|---|---|---|---|
| Sample 5 (average299, 59 g/m²) | init (mm) | 24,85 | 23,61 | 21,33 | 19,89 | 15,91 | 11,79 | 9,36 |
| | final (mm) | 19,63 | 18,03 | 14,76 | 12,83 | 8,14 | 5,62 | 4,57 |
| | **point %** | **79,00** | **76,36** | **69,18** | **64,53** | **51,14** | **47,69** | **48,76** |
| Sample 6 (average 306,40 g/m²) | init (mm) | 25,17 | 24, 30 | 22,32 | 20,92 | 16,79 | 12,40 | 9,86 |
| | final (mm) | 19,94 | 18,45 | 15,21 | 13,38 | 8,89 | 6,06 | 4,75 |
| | **point %** | **79,22** | **75,92** | **68,17** | **63,95** | **52,96** | **48,87** | **48,11** |
| Sample 7 (average 305,56 g/m²) | init (mm) | 21,83 | 20,28 | 18,11 | 17,07 | 14,15 | 11,33 | 9,11 |
| | final (mm) | 17,14 | 15,65 | 13,02 | 11,36 | 8,10 | 5,62 | 4,41 |
| | **point %** | **78,55** | **77,20** | **71,86** | **66,55** | **57,23** | **49,59** | **48,37** |
| Sample 8 (average 309,39 g/m²) | init (mm) | 24,53 | 23,49 | 21,60 | 20,26 | 16,45 | 12,49 | 9,97 |
| | final (mm) | 19,42 | 17,77 | 14,76 | 13,21 | 8,97 | 5,98 | 4,65 |
| | **point %** | **79,18** | **75,64** | **68,34** | **65,17** | **54,56** | **47,86** | **46,58** |
| Thermoloft 21 (average 302,15 g/m²) | init (mm) | 26,10 | 25,93 | 23,00 | 21,30 | 17,51 | 13,53 | 10,81 |
| | final (mm) | 20,15 | 18,73 | 15,82 | 13,77 | 9,46 | 6,58 | 4,97 |
| | **point %** | **77,18** | **72,23** | **68,78** | **64,67** | **54,03** | **48,60** | **46,02** |

Table 5.

| | | Tensile strength (N/10cm) | Elongation at Fmax (%) | Elongation at 100 N (%) |
|---|---|---|---|---|
| Sample 5 | MD | 134,38 | 34,27 | 22,28 |
| | CD | 81,76 | 59,15 | |
| sample 6 | MD | 136,80 | 30,65 | 16,50 |
| | CD | 86,38 | 59,26 | |
| sample 7 | MD | 139,51 | 31,74 | 17,66 |
| | CD | 82,89 | 58,56 | |
| sample 8 | MD | 144,13 | 30,48 | 15,55 |
| | CD | 95,11 | 53,60 | |
| Thermoloft 21 | MD | 142,37 | 27,54 | 13,75 |
| | CD | 89,03 | 53,52 | |

Tests 2 and 3 - Thermobonded nonwoven material for use in hygiene articles

The fibers obtained in examples 1 to 3 are used to prepare nonwovens for hygiene, and in particular for use as ADL and/or absorbing cores to be incorporated in absorbing articles. These nonwovens are compared to the standard product DW T161 AF manufactured by TWE for ADL. The specifications of this material are given in table 2 below along with the characteristics of the nonwovens obtained in tests 2 and 3.

In test 2, a blend of fibers was prepared using 50 % of the fibers of example 3 of Table 1 with 50% of melting bico-fibers . In test 3, a blend of fibers was prepared using 50 % of the fibers of examples 1 and 2 (mixed) of Table 1 with 50% of melting bico-fibers.

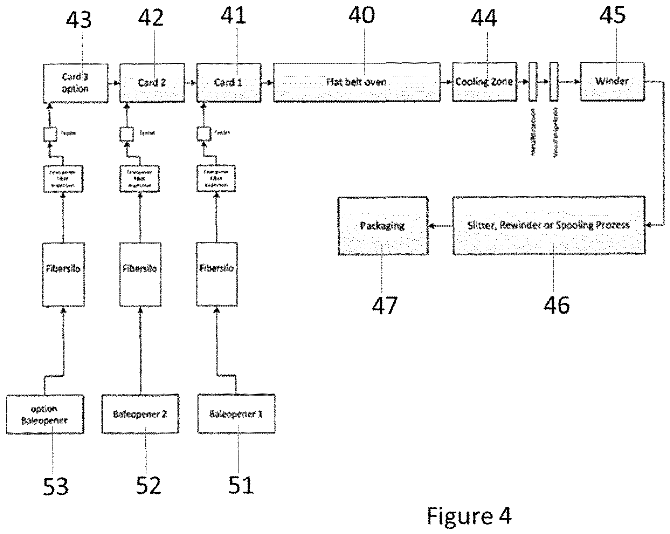

The nonwovens were manufactured in a standard manner (i.e. using one, two or three carding machines and thermobonding, as for example illustrated on figure 4.

Figure 4 illustrates a standard ADL production line comprising three carding machines 41, 42 and 43, the use of the third machine 43 being here optional. Each carding machine is respectively linked to a bale opener 51, 52 and 53 for providing the fibers (each carding machine can be linked to several bale opener nt represented for clarity purpose). Here, a fiber silo and an inspection unit are provided between each bale opener and its corresponding carding machine, as usual in the art. The webs produced by the carding machines 41, 42 and optionally 43 are superimposed before entering into the flat belt oven 40 to be bonded into a nonwoven. The nonwoven then undergoes cooling in the cooling zone 44, which is followed by an inspection step before being rolled in the winder 45. The nonwoven roll is further brought to the desired size or adjusted in step 46 before being packaged in step 47.

The Physical characteristics of the obtained nonwovens were evaluated according to the Standard Edana procedures and are given in Table 6.

The absorbing characteristics of the nonwovens of tests 2 and 3 were also measured according to the standard Edana procedures, and are given in Table 7.

The characteristics given in table 6 and 7 have been measured according to the standard method given by Edana unless otherwise stated.

As can be observed from Table 6, the nonwovens made from recycled PFS have properties falling under the specification range for the equivalent nonwoven made from virgin fibers. The opacity in the same range and the tensile strength is above the average expected value.

Table 6.

| Properties | Unit | | Test 2 | | Test 3 | | SPEIFICATIONS DW T161 AF 50gsm | | |
|---|---|---|---|---|---|---|---|---|---|
| | | | average | stdev | average | stdev | average | min | max |
| Total Weight | [g/m$^2$] | | 49,74 | 2,23 | 50,88 | 2,20 | 50,00 | 39,00 | 61,00 |
| Thickness 0.5kPa | [mm] | | 0,62 | 0,05 | 0,67 | 0,06 | | | |
| Thickness 2kPa | [mm] | | 0,47 | 0,04 | 0,49 | 0,03 | | | |
| Thickness 10kPa | [mm] | | 0,37 | 0,03 | 0,37 | 0,03 | | | |
| Tensile strength | [N/5cm] | MD | 44,39 | 3,59 | 42,41 | 3,83 | 38,00 | 20,00 | 56,00 |
| | | CD | 5,66 | 0,55 | 5,98 | 0,82 | | | |
| Elongation at Fmax | [%] | MD | 20,86 | 2,12 | 19,36 | 1,91 | | | |
| | | CD | 57,75 | 5,59 | 61,92 | 7,43 | | | |
| Elongation at 10 N | [%] | MD | 2,39 | 0,83 | 1,68 | 0,47 | | | 10,00 |
| | | CD | 0,00 | 0,00 | 0,00 | 0,00 | | | |
| Opacity (%) | | | 27,73 | | 30,25 | | 31,80 | | |

The Tensile Strength is the maximum tensile stress expressed in force per unit cross sectional area of the unstrained specimen e.g. Newtons per square millimeter, as measure according to Edana standard 110.4.R0 (15).

| Properties | Unit | | Test 2 | | Test 3 | | SPECIFICATIONS DW T161 50gsm | | |
|---|---|---|---|---|---|---|---|---|---|
| | | | average | stdev | average | stdev | average | min | max |
| Total Weight | [g/m2] | | 49,74 | 2,23 | 50,88 | 2,20 | 50,00 | 39,00 | 61,00 |
| Thickness 0.5kPa | [mm] | | 0,62 | 0,05 | 0,67 | 0,06 | | | |
| Thickness 2kPa | [mm] | | 0,47 | 0,04 | 0,49 | 0,03 | | | |
| Thickness 10kPa | [mm] | | 0,37 | 0,03 | 0,37 | 0,03 | | | |
| Tensile strength | [N/5cm] | MD | 44,39 | 3,59 | 42,41 | 3,83 | 38,00 | 20,00 | 56,00 |
| | | CD | 5,66 | 0,55 | 5,98 | 0,82 | | | |
| Elongation at Fmax | [%] | MD | 20,86 | 2,12 | 19,36 | 1,91 | | | |
| | | CD | 57,75 | 5,59 | 61,92 | 7,43 | | | |
| Elongation at 10 N | [%] | MD | 2,39 | 0,83 | 1,68 | 0,47 | | | 10,00 |
| | | CD | 0,00 | 0,00 | 0,00 | 0,00 | | | |
| Opacity (%) | | | 27,73 | | 30,25 | | 31,80 | | |
| Strike Through | [s] | | 0,90 | 0,14 | 1,14 | 0,19 | | | 2,80 |
| | | | | | | | | | |
| Wet Back | [g] | | 0,11 | 0,01 | 0,10 | 0,01 | | | 0,25 |
| Repeated ST - 1 Saline | [s] | | 0,23 | 0,10 | 0,48 | 0,13 | | | |
| Repeated ST - 2 Saline | [s] | | 0,95 | 0,10 | 1,28 | 0,15 | | | |
| Repeated ST - 3 Saline | [s] | | 0,97 | 0,11 | 1,43 | 0,14 | | | |
| Wet back after rep ST Saline | [g] | | 0,12 | 0,01 | 0,11 | 0,01 | | | |
| Run off - 1 | [g] | | 0,00 | 0,00 | 0,00 | 0,00 | | | 0,00 |
| Run off - 2 | [g] | | 0,00 | 0,00 | 0,00 | 0,00 | | | 0,00 |
| Run off - 3 | [g] | | 0,00 | 0,00 | 0,00 | 0,00 | | | 0,00 |

The Thickness is measured while applying various pressures on the material, according to Edana assay WSP 120.6.R0 (15).

The Elongation was measured according to Edana assay WSP 110.4.R0 (15) .

The opacity is measured according to the method described above.

Table 7.

| Properties | Unit | | Test 2 | | Test 3 | | SPECIFICATIONS DW T161 50gsm |
|---|---|---|---|---|---|---|---|
| | | | average | stdev | average | stdev | max |
| Strike Through | [s] | | 0,90 | 0,14 | 1,14 | 0,19 | 2,80 |

(continued)

| Properties | Unit | Test 2 average | stdev | Test 3 average | stdev | SPECIFICATIONS DW T161 50gsm max |
|---|---|---|---|---|---|---|
| Wet Back | [g] | 0,11 | 0,01 | 0,10 | 0,01 | 0,25 |
| Repeated ST - 1 *Saline* | [s] | 0,23 | 0,10 | 0,48 | 0,13 | |
| Repeated ST - 2 *Saline* | [s] | 0,95 | 0,10 | 1,28 | 0,15 | |
| Repeated ST - 3 *Saline* | [s] | 0,97 | 0,11 | 1,43 | 0,14 | |
| Wet back after rep ST *Saline* | [g] | 0,12 | 0,01 | 0,11 | 0,01 | |
| Run off - 1 | [g] | 0,00 | 0,00 | 0,00 | 0,00 | 0,00 |
| Run off - 2 | [g] | 0,00 | 0,00 | 0,00 | 0,00 | 0,00 |
| Run off - 3 | [g] | 0,00 | 0,00 | 0,00 | 0,00 | 0,00 |

The Strike Through Time is the time taken for a known volume of liquid (e.g. simulated urine) applied to the surface of a test piece of nonwoven coverstock, which is in contact with an underlying standard absorbent pad, to pass through the nonwoven. It was measured using EDANA assay WSP 70.3.R1 (19). the repeated strike through time was measured according to EDANA assay WSP 70.7.R1 (19).

The Wet Back Test intends to examine the ability of a diaper coverstock to resist the transport back onto the skin of a liquid that had already penetrated the coverstock. It was measured according to EDANA assay WSP 80.10R1 (19).
The wet back after repeated strike through (ST) was measured according to Edana assay WSP 70.8.R1 (19).

The Run Off quantifies the amount of liquid which runs down a nonwoven when a specified amount is poured onto the liquid at an angle. It is measured according to Edana assay WSP 080.9.R1 (19).

As can be observed from the results of tables 6 and 7, the ADL nonwovens incorporating recycled PSF meet the specifications set for the standard product. Some specifications are even exceed the performance of the reference material. In particular, the wet back and strike through properties are far below the maximum values allowed.

These are non limiting examples of fiber ratios, manufacturing technologies and applications for the fibers of the invention.

Nonwoven manufacturing technologies in which the PSF can be used are for example, but not limited to these:

- Mechanically bonded nonwovens by needling
- Mechanically bonded nonwovens by means of hydroentanglement
- Resin bonded nonwovens by means of foam bath
- Spray bonded nonwovens by means of sprayed resin
- Thermally bonded nonwovens by means of blending LMF
- Stitchbonded nonwovens,
- etc...

## Claims

1. Method for recycling polyester based nonwovens comprising 5 to 50% of PET/coPET low melt staple fibers comprising the steps of:

    - Feeding a polyester based nonwoven to recycle into a repelletizing machine to obtain recycled polyester-based pellets,
    - Blending the obtained pellets with polyester flakes, the blend comprising 15 to 35% of recycled polyester-based pellets and 65 to 85% of polyester flakes, and
    - Extruding the blend into polyester staple fibers (PSF).

2. Method according to claim 1, wherein the polyester flakes originate from recycled PET bottles.

3. Method according to claim 1 or 2, wherein the PSF contain between 0.5 and 50% of PET and/or coPET.

4. Method according to one of claim 1 to 3, further comprising the step of:

   - incorporating the PSF into a web of fibers for manufacturing a nonwoven material.

5. Method according to claim 4, further comprising the step of:

   - using the nonwoven material in an article, like for example an hygiene article, an absorbent article, an article for the automotive industry, ...

6. A polyester staple fibers (PSF) obtained by the method of one of claim 1 to 3, the PSF having an intrinsic viscosity (IV) of below 0.7 dl/g, preferably between 0.6 and 0.7 dl/g.

7. A polyester staple fibers (PSF) comprising at least 0.75% of PET and/or CoPET originating from recycled nonwovens.

8. A polyester staple fibers according to claim 7 having an intrinsic viscosity (IV) of below 0.7 dl/g.

9. Nonwoven material comprising 5 to 100% by weight of the PSF of one of claim 7 or 8 and having an opacity comprised between 20% and 50%.

10. Absorbent article comprising a topsheet forming a wearer-facing surface of the absorbent article, a backsheet and an absorbent core and/or an acquisition and distribution layer (ADL) interposed between the topsheet and the backsheet, wherein the absorbent article comprises a nonwoven material, said nonwoven material comprising between 5% and 100% by weight of PSF comprising at least 0.75% of PET and/or CoPET originating from recycled nonwovens.

11. Absorbent article according to claim 10, wherein the nonwoven comprises at least 20% by weight core/sheath bicomponent fibers.

12. Absorbent article according to one of claim 10 or 11, wherein the nonwoven is comprised at least in the ADL.

13. Absorbent article according to one of claim 10 or 11, wherein the nonwoven is comprised at least in the core, like a monolayer, dual layer or triple layer core.

14. Absorbent article according to anyone of claim 10 to 13, wherein the nonwoven is provided between the topsheet and the absorbent core.

15. Use of the nonwoven of claim 9 in an article for automotive applications, building, bedding, quilting and/or furniture applications or in a hygiene and/or medical article.

Figure 1

Figure 2

Figure 3

Figure 4

Europäisches
Patentamt

European
Patent Office

Office européen
des brevets

**EUROPEAN SEARCH REPORT**

Application Number

EP 21 16 6826

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/153643 A1 (FERRELL RICHARD FRANCIS [US] ET AL) 23 May 2019 (2019-05-23) | 1-9,15 | INV.<br>D04H1/4274 |
| Y | * paragraphs [0084], [0087]; claim 1; figure 1 * | 10-14 | D04H1/435<br>D01F6/62<br>A61F13/49 |
| X | VIKAS NADKARNI: "Polyester Waste Recycling: Sources, Processing Methods & End Uses", INTERNATIONAL FIBER JOURNAL, INTERNATIONAL MEDIA GROUP, CHARLOTTE, NC, US, vol. 14, no. 3, 1 June 1999 (1999-06-01), pages 18-24, XP002173262, ISSN: 1049-801X | 1-9,15 | |
| Y | * page 18 * | 10-14 | |
| X | Anonymous: "Recycling of PET nonwoven fibre waste", PET:nology, 1 March 2017 (2017-03-01), XP55840628, Retrieved from the Internet: URL:https://www.petnology.com/competence-online/news/recycling-of-pet-nonwoven-fibre-waste.html [retrieved on 2021-09-14] | 1-9 | |
| | | | TECHNICAL FIELDS SEARCHED (IPC) |
| Y | * the whole document * | 10-14 | D04H<br>D01F<br>A61F |
| A | ANONYMOUS: "Nonwoven materials using recycled polymer", RESEARCH DISCLOSURE, KENNETH MASON PUBLICATIONS, HAMPSHIRE, UK, GB, vol. 456, no. 85, 1 April 2002 (2002-04-01), XP007130202, ISSN: 0374-4353 * the whole document * | 1-15 | |
| A | EP 2 450 480 A1 (PROMODEFI SA [BE]) 9 May 2012 (2012-05-09) * paragraph [0001]; claims 1-3 * | 1-15 | |

-/--

The present search report has been drawn up for all claims

| Place of search | Date of completion of the search | Examiner |
|---|---|---|
| Munich | 12 October 2021 | Saunders, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.............................................................

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | TR 1999 01921 A1 (SASA DUPONT SABANCI POLYESTER [TR]) 23 December 2002 (2002-12-23) * abstract; figure 1 * ----- | 1-15 | |
| Y | WO 2019/032063 A2 (EVYAP SABUN YAG GLISERIN SANAYI VE TICARET ANONIM SIRKETI [TR]) 14 February 2019 (2019-02-14) * page 4, line 20 - page 5, line 25 * ----- | 10-14 | |

TECHNICAL FIELDS
SEARCHED      (IPC)

The present search report has been drawn up for all claims

| Place of search | Date of completion of the search | Examiner |
|---|---|---|
| Munich | 12 October 2021 | Saunders, Thomas |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt
European
Patent Office
Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 21 16 6826

The Search Division considers that the present European patent application does not comply with the requirements of unity of invention and relates to several inventions or groups of inventions, namely:

1. claims: 1-6

    Method for recycling polyester based nonwovens comprising 5 to 50% of PET/coPET low melt staple fibers comprising the steps of: (a) feeding a polyester based nonwoven to recycle into a repelletizing machine to obtain recycled polyester-based pellets, (b) blending the obtained pellets with polyester flakes, the blend comprising 15 to 35% of recycled polyester-based pellets and 65 to 85% of polyester flakes, and (c) extruding the blend into polyester staple fibers

    ---

2. claims: 7-15

    Polyester staple fibres comprising at least 0.75% PET and/or CoPET originating from recycled nonwovens.

    ---

## ANNEX TO THE EUROPEAN SEARCH REPORT
## ON EUROPEAN PATENT APPLICATION NO.

EP 21 16 6826

This annex lists the patent family members relating to the patent documents cited in the above-mentioned European search report.
The members are as contained in the European Patent Office EDP file on
The European Patent Office is in no way liable for these particulars which are merely given for the purpose of information.

12-10-2021

| Patent document cited in search report | | Publication date | Patent family member(s) | | Publication date |
|---|---|---|---|---|---|
| US 2019153643 | A1 | 23-05-2019 | NONE | | |
| EP 2450480 | A1 | 09-05-2012 | BE<br>EP | 1019138 A5<br>2450480 A1 | 06-03-2012<br>09-05-2012 |
| TR 199901921 | A1 | 23-12-2002 | NONE | | |
| WO 2019032063 | A2 | 14-02-2019 | RU<br>TR<br>WO | 2744628 C1<br>201701975 A2<br>2019032063 A2 | 12-03-2021<br>27-08-2018<br>14-02-2019 |

For more details about this annex : see Official Journal of the European Patent Office, No. 12/82

EPO FORM P0459